# EUROPÄISCHE PATENTSCHRIFT

(11) **EP 1 185 234 B1**
(45) Veröffentlichungstag und Bekanntmachung des Hinweises auf die Patenterteilung: **09.02.2005**
(21) Anmeldenummer: 00943767.4
(22) Anmeldetag: 06.06.2000
(51) Int. Cl.: A61K 7/13

(54) **NEUE KUPPLERKOMPONENTEN FÜR OXIDATIONSHAARFARBEN**
NEW COUPLING COMPONENTS FOR OXIDATION HAIR COLORANTS
AGENTS DE COUPLAGE POUR COLORATIONS DE CHEVEUX A OXYDATION

(30) Priorität: 15.06.1999 DE 19927073
(43) Veröffentlichungstag der Anmeldung: 13.03.2002
(73) Patentinhaber: Henkel Kommanditgesellschaft auf Aktien, 40589 Düsseldorf-Holthausen (DE)
(72) Erfinder: NAUMANN, Frank, D-40593 Düsseldorf (DE); ROSE, David, D-40723 Hilden (DE); HÖFFKES, Horst, D-40595 Düsseldorf (DE)
(86) Internationale Anmeldenummer: PCT/EP2000/005156
(87) Internationale Veröffentlichungsnummer: WO 2001/000152

(56) Entgegenhaltungen:
- EP-A- 0 398 702
- DE-A- 19 746 249
- GB-A- 1 486 576

## Beschreibung

Diese Erfindung betrifft Färbemittel, die als Kupplerkomponente m-Aminophenol-Derivate enthalten, sowie deren Verwendung zum Färben von Keratinfasern.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2'-hydroxyethyl)-N,N'-bis(4'-aminophenyl)-diamino-propan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Reibung und den Einfluß chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten.

Die britische Patentschrift GB 1 486 576 offenbart ein Oxidationsfärbemittel aus einer Entwicklerkomponente und einem m-Aminophenolderivat , worunter das 3-[2'-(diethylamino-ethyl-)-amino]-phenol als ein Beispiel für eine geeignete Kupplerkomponente genannt wird.

Die Patentschriften DE 197 46 249 und EP 0 398 702 beschreiben ein Oxidationsfärbemittel aus m-Aminophenol-derivaten als Kuppler und z.B. Tetraminopyrimidin als Entwickler. Darüber hinaus wird die Verwendung von Aminophenolderivaten als Kuppler zum Färben von keratinischen Fasern erwähnt.

Aufgabe der vorliegenden Erfindung war es daher, neue Kupplerkomponenten zu entwickeln, die die an Oxidationsfarbstoffvorprodukte gestellten Anforderungen erfüllen und Färbungen in einem breiten Farbspektrum mit guten Echtheitseigenschaften ermöglichen.

Es wurde nun überraschenderweise gefunden, daß Haarfärbemittel, die mindestens eine Entwicklerkomponente sowie mindestens ein m-Aminophenolderivat der Formel (I) als Kupplerkomponente enthalten, die gestellten Aufgaben in hervorragender Weise lösen.

Daher betrifft die vorliegende Erfindung Oxidationsfärbemittel zum Färben von Keratinfasern, insbesondere menschlichen Haaren, die in einem zum Färben geeigneten Medium mindestens eine Entwicklerkomponente und mindestens ein m-Aminophenolderivat der Formel (I) enthalten 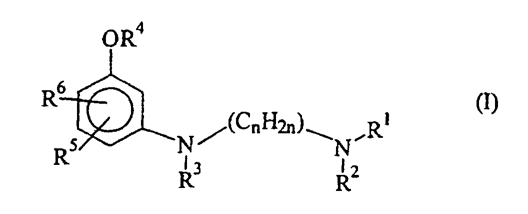 worin R¹ und R² für Wasserstoff als auch R³, R⁴, R⁵ und R⁶ für Wasserstoff stehen und n eine ganze Zahl von 1 bis 8 ist, mit der Maßgabe, daß, wenn ein Tetraaminopyrimidin als Entwicklerkomponenten eingesetzt wird, mindestens ein weiteres Farbstoffvorprodukt vorhanden ist.

Unter Keratinfasem sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Besonders bevorzugt im Sinne der vorliegenden Erfindung sind Oxidationsfärbemittel in einem wäßrigen Träger oder in Pulverform.

Beispiele für die als Substituenten in den erfindungsgemäßen Verbindungen genannten, C₁- bis C₄-Alkylgruppen sind die Gruppen Methyl, Ethyl, Propyl, Isopropyl und Butyl. Ethyl und Methyl sind bevorzugte Alkylgruppen. Eine erfindungsgemäß bevorzugte C₁bis C₄-Alkoxygruppe, ist beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Beispiele für ein Halogenatom sind erfindungsgemäß ein F-, ein Cl- oder ein Br-Atom, ein Cl-Atom ist ganz besonders bevorzugt. Beispiele für die von R¹ und R² gemeinsam mit dem Stickstoffatom, an das sie gebunden sind, gebildeten, gesättigten heterocyclischen 5- oder 6-gliedrigen Ringe sind Morpholin. Piperidin und Pyrrolidin.

Mit den erfindungsgemäßen Kupplerkomponenten können zumeist hellgelbe bis dunkelbraune Färbungen erzeugt werden, die sich insbesondere durch ihre erhöhte Wasch- und Lichtechtheit auszeichnen. Somit stellen diese Kupplerkomponenten eine wertvolle Bereicherung der Oxidationsfarbstoffvorprodukte dar.

In der DE-24 47 017 werden Haarfärbemittel beschrieben, die ein Tetraaminopyrimidin als Entwicklerkomponente sowie m-Aminophenolderivate als Blaukuppler enthalten. Obwohl die Kupplerkomponenten der vorliegenden Erfindung unter die breite allgemeine Formel dieser Blaukuppler fallen, ist den eher vagen Ausführungen keinerlei Hinweis auf die hervorragenden färberischen Eigenschaften der erfindungsgemäßen Kuppler auch in anderen Bereichen des Farbspektrums zu entnehmen.

Färbungen, die mit einer Zweierkombination einer Kupplerkomponente der Formel (I) mit Tetraaminopyrimidin oder einem seiner Derivate als Entwicklerkomponente erhalten werden, weisen nur schlechte Echtheitseigenschaften auf, da sie dazu neigen, sich im Laufe der Zeit zu verändern. Um diese Farbveränderungen zu vermeiden, müssen die erfindungsgemäßen Färbemittel in diesen Fällen zwingend mindestens ein weiteres Farbstoffvorprodukt vom Typ Kuppler oder Entwickler enthalten. Zu diesem Zwecke bevorzugte Entwickler- beziehungsweise Kupplerkomponenten können den folgenden Ausführungen entnommen werden.

Die Herstellung der erfindungsgemäßen Kuppler ist teilweise bereits in der Literatur beschrieben worden (Bull. Soc. Chim. Fr., 1947, S. 827-829). Die weiteren erfindungsgemäßen Kupplerkomponenten können mit Hilfe üblicher Syntheseverfahren hergestellt werden.

Da es sich bei allen erfindungsgemäßen Substanzen um Amino-Verbindungen handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden m-Aminophenolderivate gemäß Formel (I) als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate.

Die erfindungsgemäßen Oxidationsfärbemittel enthalten prinzipiell mindestens eine Kupplerkomponente gemäß Formel (I) sowie mindestens eine Entwicklerkomponente.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxy-ethylaminomethyl-4-amino-phenol, 4,4'-Diaminodiphenylamin, 4-Amino-3-fluorphenol, 2-Aminomethyl-4-amino-phenol, 2-Diethylaminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z.B. 4,5-Diamino-1-(2'-hydroxyethyl)-pyrazol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Hydroxy-4,5,6-triaminopyrimidin, 4-Hydroxy-2,5,6-triaminopyrimidin, 4,5-Diamino-1-(2'-hydroxyethyl)-pyrazol, 2-Aminomethyl-4-amino-phenol, 2-Diethylaminomethyl-4-amino-phenol, N,N-Bis-(2-hydroxyethyl)-p-phenylendiamin, Bis-(2-hydroxy-5-aminophenyl)-methan, N,N'-Bis-(β-hydroxyethyl)-N,N'-bis-(4-aminophenyl)-1,3-diamino-propan-2-ol und o-Aminophenol.

Zur Nuancierung können die erfindungsgemäßen Färbemittel weitere Kupplerkomponenten enthalten. Erfindungsgemäß bevorzugte Kupplerkomponenten sind:
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 3-Amino-6-methoxy-2-methylaminophenol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol. 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol sowie
- Methylendioxybenzolderivate wie beispielsweise 3,4-Methylendioxyphenol, 1-Hydroxy-3,4-methylendioxybenzol, 1-Amino-3,4-methylendioxybenzol und 1-(2'-Hydroxyethyl)-amino-3,4-methylendioxybenzol.

Weitere Kupplerkomponenten sind ferner 3-Dimethylaminophenol, 2,4-Dihydroxyanilin, 8-Amino-6-methoxy-chinolin, 2-Amino-5-naphthol-1,7-disulfonsäure, 3-Amino-1-phenyl-5-pyrazolon, 3,5-Diaminobenzamid, 3-Methylsulfonylamino-2-methylanilin, 5,6-Dihydroxybenzimidazol, 2,2'-Dihydroxybenzylamin, 3,5,3',5'-Tetraamino-2,2'-dimethoxy-diphenyl, 3,5-Diamino-pchlorbenzotrifluorid, 4-Methyl-3-aminophenol, 2,4-Diamino-3-chlorphenol, 1-Amino-3-di-(2-hydroxyethylamino)-4-ethoxybenzol, 2,4-Dimethylresorcin, Bis-(2,4-diaminophenoxy)-methan, 2,6-Bis-(hydroxyethyl)-pyridin, 4-Hydroxy-3-methoxybenzylalkohol, 8-Hydroxychinolin, 4-Hydroxy-3-methoxy-benzylamin, 4-Ethylresorcin, 2-Methylthio-5-aminophenol, 5-[(3-Hydroxypropyl)amino]-2-methylphenol, 2,6-Dimethoxy-3-aminophenol und 2,6-Diamino-3-methylthiotoluol.

Besonders bevorzugte weitere Kupplerkomponenten sind 1-Naphthol, 1,5-, 2,7- und 1,7-Dihydroxynaphthalin, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 6-Methyl-1,2,3,4-tetrahydrochinoxalin, m-Aminophenol, o-Aminophenol und 2-Chlorresorcin.

Diese weiteren Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet I, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-Ihydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Färbemittel können die Oxidationsfarbstoffvorprodukte in einen geeigneten wasserhaltigen Träger eingearbeitet werden. Solche Träger sind zum Zwecke der Haarfärbung z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropion-säuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsar-cosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammo-niumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkyl-ammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere, quaternierter Polyvinylalkohol und Polyquaternium-2
- zwitterionische und amphotere Polymere wie beispielsweise Acrylamidopropyl-trimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmnittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme (Enzymklasse 1: Oxidoreduktasen) Elektronen aus geeigneten Entwicklerkomponenten (Reduktionsmittel) auf Luftsauerstoff übertragen. Bevorzugt wären Oxidasen wie Tyrosinase und Laccase aber auch Glucoseoxidase, Uricase oder Pyruvatoxidase. Weiterhin können die Enzyme zur Verstärkung der Wirkung geringer Mengen vorhandener Oxidationsmittel dienen. Ein Beispiel für ein derartiges enzymatisches Verfahren stellt das Vorgehen dar, die Wirkung geringer Mengen (z.B. 1% und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein zweiter Gegenstand dieser Erfindung ist die Verwendung der vorgenannten Mittel zum Färben keratinischer Fasern.

Die nachfolgenden Beispiele sollen den Erfindungsgegenstand näher erläutern.

### Ausführungsbeispiele

### 1. Herstellung der Färbecreme

### 1.1 Teilmischung A

| | |
|---|---|
| Hydrenol® D¹ | 8,50g |
| Lorol® techn.² | 2,00g |
| Eumulgin® B2³ | 0,75g |
| Texapon® NSO⁴ | 20,00g |
| Dehyton® K⁵ | 12,50g |
| Wasser | 30,00g |

| | |
|---|---|
| ¹ C₁₆₋₁₈-Fettalkohol (INCI-Bezeichnung: Cetearyl alcohol) (HENKEL) | |
| ² C₁₂₋₁₈-Fettalkohol (INCI-Bezeichnung: Coconut alcohol) (HENKEL) | |
| ³ Cetylstearylalkohol mit ca. 20 EO-Einheiten (INCI-Bezeichnung: Ceteareth-20) (HENKEL) | |
| ⁴ Laurylethersulfat, Natriumsalz (ca. 27,5% Aktivsubstanz; INCI-Bezeichnung: Sodium Laureth Sulfate) (HENKEL) | |
| ⁵ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (HENKEL) | |

Die Substanzen Hydrenol® D, Lorol® und Eumulgin® B2 wurden bei 80°C aufgeschmolzen, mit dem 80°C heißem Wasser, enthaltend Texapon® NSO und Dehyton® K, vermischt und unter starkem Rühren emulgiert. Danach wurde die Emulsion unter schwachem Rühren abgekühlt.

### 1.2 Teilmischung B

| | |
|---|---|
| Natriumsulfit | 1,00g |
| Ammoniumsulfat | 1,00g |
| Farbstoffvorprodukte | jeweils 2,5mmol |
| Ammoniak (25%ige Lösung) | ad pH=10,0 |
| Wasser | 10,00g |

Die Farbstoffvorprodukte wurden in dem 50°C heißem Wasser unter Zugabe von Natriumsulfit, Ammoniumsulfat und Ammoniak gelöst.
Die Farbstoffvorproduktlösung (Teilmischung B) wurde zur Emulsion (Teilmischung A) gegeben, mit Ammoniak auf pH=10 eingestellt und mit Wasser auf 100 g aufgefüllt. Es wurde bis zum Erreichen der Raumtemperatur weitergerührt.

### 2. Färbung der keratinischen Fasern

Die so erhaltene Färbecreme wurde im Verhältnis 1:1 mit einer 1%-igen H₂O₂-Lösung vermischt und auf 5cm lange Strähnen standardisierten nicht besonders vorbehandelten Menschenhaares (Kerling) aufgetragen. Nach 30min Einwirkzeit bei 32°C wurde das Haar gespült, mit einem üblichen Haarwaschmittel ausgewaschen und anschließend getrocknet.

Die Ergebnisse der Färbeversuche sind den folgenden Tabellen zu entnehmen.

Die dabei erhaltenen Färbungen sind nachstehender Tabelle I zu entnehmen.

**Tabelle I:**

| Ausfärbungen | | |
|---|---|---|
| Kuppler | Entwickler | Nuance der gefärbten Strähne |
| 3-(2'-Aminoethyl)-aminophenol | p-Toluylendiamin | Dunkelbraun |
| 3-(2'-Aminoethyl)-aminophenol | p-Aminophenol | Topasgelb |
| 3-(2'-Aminoethyl)-aminophenol | 1-(2'-Hydroxyethyl)-2,5-diaminobenzol | Graubraun |
| 3-(2'-Aminoethyl)-aminophenol | 4-Amino-3-methylphenol | Hellgelb |
| 3-(2'-Aminoethyl)-aminophenol | 4-Amino-2-aminomethylphenol | Topasgelb |
| 3-(2'-Aminoethyl)-aminophenol | 4-Hydroxy-2,5,6-triaminopyrimidin | Rotbraun |
| 3-(3'-Aminopropyl)-aminophenol | p-Toluylendiamin | Graubraun |
| 3-(3'-Aminopropyl)-aminophenol | N,N'-Bis-(β-hydroxyethyl)-N,N'-bis-(4-aminophenyl)-1,3-diaminopropan-2-ol | Schwarzblau Schwarzblau |
| 3-(3'-Aminopropyl)-aminophenol | p-Aminophenol | Topasgelb |
| 3-(3'-Aminopropyl)-aminophenol | 4-Amino-2-aminomethylphenol | Braun |
| 3-(3-Aminopropyl)-aminophenol | 4-Hydroxy-2,5,6-triaminopyrimidin | Dunkelmagenta |
| 3-(3'-Aminopropyl)-aminophenol | 1-(2'-Hydroxyethyl)-2,5-diaminobenzol | Schokoladenbraun |
| 3-(3'-Aminopropyl)-aminophenol | 4-Amino-3-methylphenol | Goldblond |

Den Tabellen III und IV sind weitere erfindungsgemäße Färbemittel zu entnehmen. Die Mengenangaben sind, sofern nichts anderes vermerkt ist, in Gewichtsprozent angegeben.

**Tabelle III:**

| **Rohstoff** | **1** | **2** | **3** | **4** |
|---|---|---|---|---|
| Fettalkoholgemisch C₁₂-C₁₈ | 10,5 | 10,5 | 10,5 | 10,5 |
| Eumulgin® B 2 | 1,0 | 1,0 | 1,0 | 1,0 |
| Texapon® NSO | 12,5 | 12,5 | 12,5 | 12,5 |
| Dehyton® K | 6,5 | 6,5 | 6,5 | 6,5 |
| Polymer JR® ⁶ | 0,8 | 0,8 | 0,8 | 0,8 |
| Natriumsulfit | 0,3 | 0,3 | 0,3 | 0,3 |
| Ammoniumsulfat | 0,5 | 0,5 | 0,5 | 0,5 |
| 3-[(2'-Aminoethyl) amino] phenol | 1,07 | 0,96 | 0,277 | 0,03 |
| p-Toluylendiaminsulfat | -- | -- | 0,27 | 0,80 |
| 1-(2'-Hydroxyethyl)-2,5-diaminobenzol-sulfat | -- | -- | -- | 0,13 |
| p-Phenylendiamin-hydrochlorid | -- | -- | 0,25 | -- |
| p-Aminophenol | -- | -- | -- | 0,01 |
| 3-Methyl-4-aminophenol | -- | -- | 0,36 | -- |
| Bis-(2-hydroxy-5-aminophenyl) methan-dihydrochlorid | -- | -- | 0,37 | -- |
| 2-Aminomethyl-p-amino-phenol-dihydrochlorid | -- | -- | 0,25 | -- |
| 2,4,5,6-Tetraaminopyrimidin-sulfat | 3,33 | -- | - | -- |
| 4-Hydroxy-2,5,6-triaminopyrimidin-sulfat | -- | 3,00 | -- | -- |
| Resorcin | -- | -- | 0,15 | 0,03 |
| 2-Methylresorcin | -- | -- | 0,17 | 0,03 |
| 4-Chlorresorcin | -- | -- | -- | 0,02 |
| 2,7-Dihydroxynapthalin | -- | -- | -- | 0,08 |
| 2-Methylamino-3-amino-6-methoxypyridin | 1,58 | 0,71 | -- | 0,003 |
| 1,3-Bis-(2,4-diamino-phenoxy) propan-tetra-hydrochlorid | -- | -- | -- | 0,002 |
| 2,6-Dihydroxy-3,4-dimethylpyridin | -- | -- | -- | 0,10 |
| 1,5-Dihydroxy napthalin | -- | -- | -- | 0,05 |
| 5-Amino-2-methylphenol | -- | -- | 0,17 | 0,20 |
| 3-Amino-2-chlor-6-methylphenol | -- | -- | -- | 0,09 |
| 2-Amino-3-hydroxy-pyridin | -- | -- | 0,05 | -- |
| 2,6-Dimethoxy-3,5-diaminopyridin | -- | 0,76 | -- | -- |

| | | | | |
|---|---|---|---|---|
| ⁶ quaternierte Hydroxyethylcellulose (INCI-Bezeichnung: Polyquatemium-10) (Amerchol) | | | | |

**Tabelle IV:**

| **Rohstoff** | **5** | **6** | **7** |
|---|---|---|---|
| Fettalkoholgemisch C₁₂-C₁₈ | 10,5 | 10,5 | 10,5 |
| Eumulgin® B2 | 1,0 | 1,0 | 1,0 |
| Texapon® NSO | 12,5 | 12,5 | 12,5 |
| Dehyton® K | 6,5 | 6,5 | 6,5 |
| Polymer JR® | 0,8 | 0,8 | 0,8 |
| Natriumsulfit | 0,3 | 0,3 | 0,3 |
| Ammoniumsulfat | 0,5 | 0,5 | 0,5 |
| 3-[(2'-Aminoethyl) amino] phenol | 0,12 | 0,01 | 0,16 |
| p-Toluylendiaminsulfat | 0,10 | 0,04 | 1,45 |
| p-Phenylendiamin-hydrochlorid | -- | -- | 0,50 |
| 3-Methyl-4-aminophenol | -- | -- | 0,07 |
| Bis-(2-hydroxy-5-aminophenyl) methan-dihydrochlorid | -- | -- | 0,10 |
| 2-Aminomethyl-4-amino-phenol-dihydrochlorid | -- | -- | 0,07 |
| 2,4,5,6-Tetraaminopyrimidin-sulfat | 1,8 | -- | -- |
| 4-Hydroxy-2,5,6-triaminopyrimidin-sulfat | 0,2 | -- | -- |
| Resorcin | 0,15 | -- | 0,33 |
| 2-Methylresorcin | 0,35 | -- | 0,16 |
| 2,7-Dihydroxynapthalin | 0,30 | -- | 0,60 |
| 2,4-Diaminophenoxy-ethanol-sulfat | 0,02 | 0,01 | -- |
| 2-Amino-4-(2'-hydroxy-ethyl) amino-anisol-sulfat | 0,01 | -- | -- |
| 2-Methylamino-3-amino-6-methoxypyridin | -- | -- | 0,09 |
| 1,3-Bis-(2,4-diamino-phenoxy) propan-tetra-hydrochlorid | -- | -- | 0,03 |
| 5-Amino-2-methylphenol | 0,02 | -- | -- |
| 2-Amino-3-hydroxy-pyridin | 0,01 | -- | 0,15 |
| 3-Amino-2,4-dichlor-phenol | -- | 0,02 | -- |
| 2,6-Dimethoxy-3,5-diaminopyridin | -- | -- | -- |

Die Färbemittel der Tabellen III und IV wurden mit einer 25%igen wäßrigen Ammoniaklösung auf pH 9 eingestellt und mit Wasser auf 100 Gewichtsprozent aufgefüllt. Die resultierenden Haarfärbemittel wurden jeweils mit einer kommerziellen 6%igen Wasserstoffperoxidzubereitung (Marktprodukt Poly Color Brillance) im Verhältnis 1:1 verdünnt und anschließend auf eine Haarsträhne (Kerling, naturweiß) aufgetragen. Abweichend davon wurde Beispiel 6 mit einer 12 %igen wäßrigen Wasserstoffperoxidlösung ausgefärbt. Nach 30min Einwirkzeit bei 32°C wurden die Strähnen gespült und getrocknet. Es wurden die folgenden Nuancierungen erzielt:

| Rezeptur-Nr. | Nuance |
|---|---|
| 1 | Schwarz |
| 2 | rötliches Schwarzbraun |
| 3 | dunkle Kastanie |
| 4 | Aubergine |
| 5 | rötliches Mittelbraun |
| 6 | helles Rauchblau |
| 7 | warmes Dunkelbraun |

## Patentansprüche

1. Oxidationsfärbemittel zum Färben von Keratinfasern, insbesondere menschlichen Haaren, **dadurch gekennzeichnet, daß** es in einem zum Färben geeigneten Medium mindestens eine Entwicklerkomponente und als kupplerkomponente mindestens ein m-Aminophenolderivat der Formel (I) 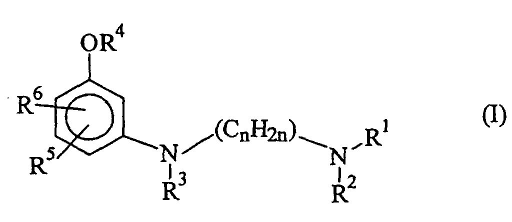 worin R¹ und R² für Wasserstoff R³, R⁴, R⁵ und R⁶ für Wasserstoff stehen und n eine ganze Zahl von 1 bis 8 ist, oder eines der physiologisch verträglichen Salze dieser Verbindungen enthält, mit der Maßgabe, daß, wenn ein Tetraaminopyrimidin als Entwicklerkomponente eingesetzt wird, mindestens ein weiteres Farbstoffvorprodukt vorhanden ist.

2. Mittel nach Anspruch 1, **dadurch gekennzeichnet, daß** die Verbindung der Formel (I) 3-(2'-Aminoethyl)-aminophenol oder 3-(3'-Aminopropyl)aminophenol ist.

3. Mittel nach einem der Ansprüche 1 oder 2, **dadurch gekennzeichnet, daß** die Entwicklerkomponente, ausgewählt ist aus der Gruppe, die aus p-Phenylendiamin, p-Toluylendiamin, p-Aminophenol, 1-(2'-Hydroxyethyl)-2,5-diaminobenzol, 4-Amino-3-methylphenol, 2-Hydroxy-4,5,6-triaminopyrimidin, 4-Hydroxy-2,5,6-triaminopyrimidin, 4,5-Diamino-1-(2-hydroxyethyl)-pyrazol, 2-Aminomethyl-4-amino-phenol, 2-Diethylaminomethyl-4-aminophenol, N,N-Bis-(2-hydroxyethyl)-p-phenylendiamin, Bis-(2-hydroxy-5-aminophenyl)-methan, N,N'-Bis-(β-hydroxyethyl)-N,N'-bis-(4-aminophenyl)-1,3-diamino-propan-2-ol und o-Aminophenol gebildet wird.

4. Mittel nach einem der Ansprüche 1 bis 3, **dadurch gekennzeichnet, daß** es mindestens eine weitere Kupplerkomponente ausgewählt aus der Gruppe, die gebildet wird von 1-Naphthol, 1,5-, 2,7- und 1,7-Dihydroxynaphthalin, 5-Amino-2-methylphenol, 2-Amino-3-Hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 6-Methyl-1,2,3,4-tetrahydrochinoxalin, m-Aminophenol, o-Aminophenol und 2-Chlorresorcin, enthält.

5. Mittel nach einem der Ansprüche 1 bis 4, **dadurch gekennzeichnet, daß** Entwicklerkomponenten in einer Menge von 0,005 bis 20 Gew.-%, vorzugsweise 0,1 bis 5 Gew.-%, und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

6. Mittel nach einem der Ansprüche 1 bis 5, **dadurch gekennzeichnet, daß** mindestens ein direktziehender Farbstoff enthalten ist.

7. Verwendung von Mitteln nach einem der Ansprüche 1 bis 6 zum Färben keratinischer Fasern.

## Claims

1. Oxidation colorant for colouring keratin fibres, more particularly human hair, **characterized in that** it contains at least one primary intermediate and - as secondary intermediate - at least one m-aminophenol derivative corresponding to formula (I): 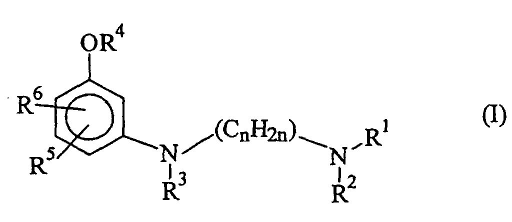 in which R¹ and R² represent hydrogen, R³, R⁴, R⁵ and R⁶ represent hydrogen and n is an integer of 1 to 8,
or a physiologically compatible salt of these compounds in a medium suitable for colouring, with the proviso that, where a tetraaminopyrimidine is used as the primary intermediate, at least one other dye precursor is present.

2. Colorant as claimed in claim 1, **characterized in that** the compound of formula (I) is 3-(2'-aminoethyl)-aminophenol or 3-(3'-aminopropyl)-aminophenol.

3. Colorant as claimed in claim 1 or 2, **characterized in that** the primary intermediate is selected from the group consisting of p-phenylenediamine, p-toluylenediamine, p-aminophenol, 1-(2'-hydroxyethyl)-2,5-diaminobenzene, 4-amino-3-methylphenol, 2-hydroxy-4,5,6-triaminopyrimidine, 4-hydroxy-2,5,6-triaminopyrimidine, 4,5-diamino-1-(2-hydroxyethyl)-pyrazole, 2-aminomethyl-4-amino-phenol, 2-diethylaminomethyl-4-amino-phenol, N,N-bis-(2-hydroxyethyl)-p-phenylenediamine, bis-(2-hydroxy-5-aminophenyl)-methane, N,N'-bis-(β-hydroxyethyl)-N,N'-bis-(4-aminophenyl)-1,3-diaminopropan-2-ol and o-aminophenol.

4. Colorant as claimed in any of claims 1 to 3, **characterized in that** it contains at least one other secondary intermediate selected from the group consisting of 1-naphthol, 1,5-, 2,7- and 1,7-dihydroxynaphthalene, 5-amino-2-methylphenol, 2-amino-3-hydroxypyridine, resorcinol, 4-chlororesorcinol, 2-chloro-6-methyl-3-aminophenol, 2-methylresorcinol, 5-methylresorcinol, 2,5-dimethylresorcinol, 2,6-dihydroxy-3,4-dimethylpyridine, 6-methyl-1,2,3,4-tetrahydroquinoxaline, m-aminophenol, o-aminophenol and 2-chlororesorcinol.

5. Colorant as claimed in any of claims 1 to 4, **characterized in that** it contains primary intermediates in a quantity of 0.005 to 20% by weight and preferably 0.1 to 5% by weight and secondary intermediates in a quantity of 0.005 to 20% by weight and preferably 0.1 to 5% by weight, based on the oxidation colorant as a whole.

6. Colorant as claimed in any of claims 1 to 5, **characterized in that** it contains at least one substantive dye.

7. The use of the colorants claimed in any of claims 1 to 6 for colouring keratinous fibres.

## Revendications

1. Agent de teinture par oxydation pour teindre des fibres kératiniques, en particulier des cheveux humains, **caractérisé en ce qu'**il contient dans un milieu convenant à la coloration, au moins un composant révélateur et en tant que composant de couplage, au moins un dérivé m-aminophénolique de formule (I) 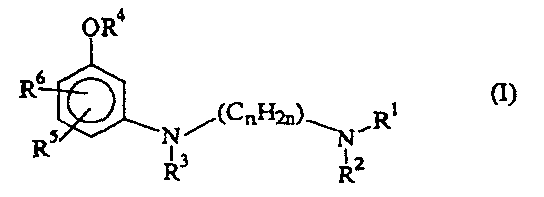 dans laquelle R¹ et R² représentent un atome d'hydrogène, R³, R⁴, R⁵ et R⁶ représentent un atome d'hydrogène, et n est un nombre entier de 1 à 8, ou l'un des sels acceptables sur le plan physiologique de ces composés, à condition que, lorsqu'une tétraaminopyrimidine est mise en oeuvre en tant que composant révélateur, au moins un produit supplémentaire précurseur de colorant soit présent.

2. Agent selon la revendication 1, **caractérisé en ce que** le composé de formule (I) est le 3-(2'-aminoéthyl)-aminophénol, ou le 3-(3'-aminopropyl)aminophénol.

3. Agent selon l'une quelconque des revendications 1 ou 2, **caractérisé en ce que**, le composant révélateur est choisi dans le groupe qui est formé par la p-phénylènediamine, la p-toluylènediamine, le p-aminophénol, le 1-(2'-hydroxyéthyl)-2,5-diaminobenzène, le 4-amino-3-méthylphénol, la 2-hydroxy-4,5,6-triaminopyrimidine, la 4-hydroxy-2,5,6-triaminopyrimidine, le 4,5-diamino-1-(2-hydroxyéthyl)-pyrazole, le 2-aminométhyl-4-aminophénol, le 2-diéthylaminométhyl-4-aminophénol, la N,N-bis-(2-hydroxyéthyl)-p-phénylène-diamine, le bis-(2-hydroxy-5-aminophényl)méthane, le N,N'-bis-(β-hydroxy-éthyl)-N,N'-bis-(4-aminophényl)-1,3-diamino-propan-2-ol et l'o-aminophénol.

4. Agent selon l'une quelconque des revendications 1 à 3, **caractérisé en ce qu'**il contient au moins un composant de couplage supplémentaire, choisi dans le groupe qui est formé par le 1-naphtol, le 1,5-, 2,7- et 1,7-dihydroxynaphtalène, le 5-amino-2-méthylphénol, la 2-amino-3-hydroxypyridine, la résorcine, la 4-chlororésorcine, le 2-chloro-6-méthyl-3-aminophénol, la 2-méthylrésorcine, la 5-méthylrésorcine, la 2,5-diméthylrésorcine, la 2,6-dihydroxy-3,4-diméthylpyridine, la 6-méthyl-1,2,3,4-tétrahydroquinoxaline, le m-aminophénol, l'o-aminophénol, et la 2-chlororésorcine.

5. Agent selon l'une quelconque des revendications 1 à 4 **caractérisé en ce que**, des composants révélateurs y sont contenus en une quantité de 0,005 à 20% en poids, de préférence de 0,1 à 5% en poids, et des composants de couplage en une quantité de 0,005 à 20% en poids, de préférence de 0,1 à 5% en poids, à chaque fois par rapport à l'agent de teinture par oxydation au total.

6. Agent selon l'une quelconque des revendications 1 à 5, **caractérisé en ce qu'**au moins un colorant montant directement sur les fibres y est contenu.

7. Utilisation d'agents selon l'une quelconque des revendications 1 à 6, pour la teinture de fibres kératiniques.
